# EUROPÄISCHE PATENTSCHRIFT

(11) **EP 1 802 243 B1**
(45) Veröffentlichungstag und Bekanntmachung des Hinweises auf die Patenterteilung: **26.11.2014**
(21) Anmeldenummer: 04791719.0
(22) Anmeldetag: 20.10.2004
(51) Int. Cl.: A61B 17/74, A61B 17/72

(54) **MARKNAGEL ZUR EINFÜHRUNG IN DEN MARKRAUM EINES FEMUR**
INTRAMEDULLARY PIN FOR INSERTION INTO THE MEDULLARY SPACE OF A FEMUR
CLOU INTRAMEDULLAIRE DESTINE A ETRE INSERE DANS L'ESPACE MEDULLAIRE D'UN FEMUR

(30) Priorität: 14.10.2004 US 522568 P
(43) Veröffentlichungstag der Anmeldung: 04.07.2007
(73) Patentinhaber: Synthes GmbH, 4436 Oberdorf (CH)
(72) Erfinder: KAUP, Thomas, CH-7270 Davos Platz (CH)
(74) Vertreter: Höhfeld, Jochen
(86) Internationale Anmeldenummer: PCT/IB2004/003425
(87) Internationale Veröffentlichungsnummer: WO 2006/040612

(56) Entgegenhaltungen:
- EP-A- 0 715 832
- US-A- 4 805 607
- US-A- 5 562 667
- US-A- 6 120 504
- US-A1- 2002 103 488
- US-A1- 2003 069 581
- US-B1- 6 210 414

## Beschreibung

Die Erfindung betrifft einen Marknagel zur Einführung in den Markraum eines Femur.

Zum Stand der Technik zählt das Dokument US6461360B1. Es beschreibt im Wesentlichen einen Marknagel für die Osteosynthese. Dieser weist vor der Einführung in einen Femur an seinem distalen Ende eine Krümmung in der Sagittalebene auf, die mit der Gegenkrümmung des Femur korrespondiert. Sein proximales Ende beschreibt weitgehend eine stetige Krümmung mit konstantem Krümmungsradius in der Frontalebene.

Die US6010506 offenbart einen Hybridnagel mit unterschiedlichen Radien, die sich alle in einer Ebene erstrecken.

Die WO02089683 offenbart die Geometrie eines Nagels als Spirale (Helix). Über diese Geometrie wird erreicht, dass der Eintrittspunkt für einen antegrad eingeführten Nagel von der Trochanterspitze nach lateral verschoben werden kann. Beim Einführen des Nagels dreht sich der Nagel um ca. 90°. Das Drehen des Nagels wird im Wesentlichen durch seine Geometrie beeinflusst. Die innere Markraumwand und Spongiosa dienen hierbei als Leitstruktur.

Bei der Anwendung der reinen Spiralgeometrie ergeben sich aber Schwierigkeiten, welche durch die variierende Anatomie von Knochen hervorgerufen wird. Beim Erreichen der Endposition liegen die distalen Verriegelungslöcher nicht in lateral-medialer Ausrichtung. Zur Korrektur muss der Nagel entweder weiter eingebracht oder zurückgezogen werden. Hierdurch dreht sich der Nagel jedoch um seine Längsachse. Dies resultiert infolge in einer unerwünschten veränderten Höhe der Verriegelungsposition. Beim Erreichen der Endposition können so die Schrauben zum Verriegeln Im Femurkopf nicht zentrisch durch den Femurhals eingebracht werden. Wenn nur die Rotation korrigiert werden soll, führt dies wiederum umgekehrt zu einer Verschiebung der Impiantationstiefe des Nagels und somit zu einer unerwünschten Änderung der Höhe der Verriegelungsposition. Sollte das proximale Nagelende noch nicht vollständig im Knochen sein, muss der Nagel tiefer eingebracht werden. Dies resultiert jedoch in einer ungewünschten Fortsetzung der Rotationsbewegung. Durch diese ändern sich wieder die optimalen Positionen der Verriegelungsoptionen.

Die US-6210414 beschreibt einen Marknagel, welcher als ein Strahlrohrstück ausgebildet ist, und aus einem proximalen Nagelabschnitt, einem mittleren Abschnitt und einem geraden distalen Nagelabschnitt besteht. Die proximalen und distalen Nagelabschnitte sind mit Schraubenlöchem versehen, die in einer nicht parallelen Weise orientiert sind. Zwischen dem proximalen und dem mittleren Nagelabschnitte ist eine Knickstelle vorgesehen, wobei die Längsachse des einen Nagelabschnittes mit der Längsachse des nächsten Nagelabschnittes einen vorbestimmten Winkel einschließt.

Wünschenswert wäre jedoch in der Praxis, dass wenn der Nagel in seinem Endimplantationstiefenbereich zu liegen kommt, bevorzugt die distalen Verriegelungslöcher parallel zur Frontalebene bzw, in lateral-medialer Ausrichtung liegen sollen. Der Nagel kann dann im proximalen Bereich mit Schrauben fixiert werden, die durch den Femurhals in den Femurkopf eingebracht werden. Für diese Verriegelung muss die Implantationstiefe des Nagels sicherstellen, dass die Schrauben zentrisch durch den Femurhals geführt werden können. Der Femurhals und Kopf sind jedoch gegenüber der Frontalebene um die Längsachse des Femur rotiert. Diese Rotation wird als Anteversionswinkel beschrieben. Dies bedingt, dass der Nagel an die anatomisch wechselnden Anteversionswinkel durch Rotieren des Nagels um seine Längsachse angepasst werden muss. Hierdurch soll erreicht werden, dass die Schrauben zentrisch durch den Femurhals und mittig im Kopf platziert werden können. Im Weiteren soll das proximale Nagelende bündig mit dem umliegenden Kortex oder tiefer sein. Dies soll verhindern, dass das umliegende Gewebe durch das proximale Nagelende irritiert wird.

Es stellt sich also die Aufgabe, einen Marknagel bereitzustellen, der nicht für jeden andersartig gewachsenen Femurknochen eine angepasste Geometrie aufweisen muss, um die oben genannten Bedingungen zu erfüllen.

Zur Lösung der gestellten Aufgabe wird von der, in der Beschreibungseinleitung zitierten US-6210414 ausgegangen, welche einen Marknagel mit den Merkmalen des Oberbegriffs des Anspruchs 1 offenbart. Die erfindungsgemäße Lösung Liegt in den, im kennzeichnenden Teil des Anspruches 1 genannten, speziellen Merkmalen.

Vorteilhafte Weiterentwicklungen der erfindungsgemäßen Lösung sind in den abhängigen Ansprüchen angegeben.

Der Erfinder löst die Aufgabe mit einer Modifikation der Enden der Spiralform. Hierbei wird das proximale Ende des Nagels in einer Ebene gebogen. Das distale Ende bleibt gerade. So wird erreicht, dass der Nagel beim Erreichen seines Endimplantationstiefenbereichs aufhört, von selbst zu rotieren. Im Endimplantationstiefenbereich kann der Nagel ohne Änderung der Rotation entlang seiner Längsachse verschoben werden. Der Nagel kann trotzdem willkürlich um seine Längsachse rotiert werden ohne Änderung seiner Implantationstiefe.

Während der distale Nagelabschnitt also wenigstens teilweise überhaupt keine Krümmung aufweist, verläuft der proximale Nagelabschnitt in lateralposteriorer Richtung, wenn er durch die laterale Kompakta eines Trochanter Major in den Markraum eingeführt ist.

Durch die Änderung der Enden der Spiralform kann ein Implantat gefertigt werden, welches für eine bestimmte Gruppe von Knochen optimal funktioniert. Die Varianz der Anatomie hat keinen Einfluss auf die Funktionalität des Implantats mehr. Das Implantat kann für das Verriegeln im Knochen optimal ausgerichtet werden.

Der erfindungsgemässe Nagel weist im proximalen Bereich vorzugsweise zwei schräg zur Längsachse, zueinander parallel verlaufende und eine dritte, die beiden ersten Bohrungen kreuzende Bohrung auf. Die Besonderheit dieser Verriegelung liegt in der Kombination der Verriegelungsmöglichkeiten. Die antegrade Verriegelung bekommt im erfindungsgemässen Nagel eine besondere Bedeutung. Die neue lateralere Eröffnung für den Nagel fällt nahezu mit der Einbringrichtung der antegraden Schraube zusammen. Wenn proximal nur eine Schraube gesetzt wird, ist somit kein weiterer Hautschnitt notwendig.

Der erfindungsgemässe Nagel weist bevorzugt zwei quer und zueinander parallel verlaufende Bohrungen und eine dazwischen angeordnete, gegenüber der durch die beiden Bohrungen definierten Ebene um die Längsachse verdrehte, ebenfalls quer verlaufende Bohrung am distalen Ende auf. Die mittlere Verriegelungsschraube ist um 25° verdreht zur linken und rechten Verriegelungsschraube angeordnet.

Die Besonderheit der distalen Verriegelung liegt in der Kombination der Verriegelungsmöglichkeiten. Zusätzlich zur allgemein bekannten Standardverriegelung befindet sich eine dritte Bohrung zwischen den beiden Standard-Bohrungen. Durch das Verriegeln des Nagels mit 3 Schrauben wird eine axiale Stabilität erreicht. Dies bedeutet, dass die Position des distalen Nagelendes fixiert wird. Der Nagel kann nicht auf den Schrauben verschoben werden. Durch den 25°-Winkel der axialen Blockierschraube wird verhindert, dass die Schraube beim Einbringen wichtige Weichteile verletzt. Dies könnte z.B. eintreten, wenn die Schraube in sagittaler Richtung (90°) eingebracht wird. Die Verriegelungsschrauben befinden sich in einem Abstand von ca. 30 mm voneinander.

Zum Einbringen des Implantates wird der Nagel mit einem Zielbügel verbunden. Dieser liegt normalerweise plan auf dem Ende des Nagels. Hierdurch ergibt sich ein stufenloser Übergang und Konturschluss zwischen Nagel und Zielbügel.

Für die fachgerechte Implantation ist es von hoher Wichtigkeit, dass das Ende des Nagels eindeutig unter Zuhilfenahme eines bildgebenden Verfahrens (Röntgen) erkannt werden kann. Dies ist nach heutigem Stand der Technik nicht oder nur unzureichend möglich. Folgen einer nicht korrekten Einbringtiefe könnten folgende sein: Wenn der Nagel nicht ausreichend tief im Knochen zu liegen kommt, kann es durch das herausstehende Nagelende zu Komplikationen wie Schmerzen, Nekrose etc. kommen. Wenn der Nagel zu tief implantiert ist, kann es zum Versatz des proximalen Nagelendes kommen. Im weiteren kann es zum Einwachsen von Knochen kommen, so dass der obere Teil des ursprünglichen Einführkanals verschlossen wird. Diese Möglichkeiten erschweren die spätere Explantation des Implantates. Im weiteren besteht die Gefahr, dass die Nagelspitze in das Knie eindringt.

Lateral ist am proximalen Ende des erfindungsgemässen Nagels bevorzugt eine Schräge ausgestaltet, die den Übergang und den Konturschluss zwischen Zielbügel und Nagel unterbricht. Hierdurch ist bei einer anteriorposterioren Aufnahme (Röntgen) das Ende des Nagels einfach und eindeutig erkennbar. Dies vereinfacht die OP und führt zu einer sichereren Anwendung und kürzeren OP-Zeit. Der Nageleintrittspunkt liegt auf der laterale Fläche des Trochanter Majors. Diese Fläche ist im Besonderen bei schlanken Patienten auspalpierbar. Dies bedeutet, dass die Fläche nur mit einer dünnen Hautschicht abgedeckt ist. Durch den lateralen Eintrittspunkt des Nagels muss verhindert werden, dass durch das proximale Nagelende das Weichteilgewebe irritiert wird. Der Nutzen der Schräge besteht also auch darin, dass die Schräge sicher stellt, dass das proximale Nagelende einen konturschlüssigen Abschluss mit der lateralen Kortexwand bildet. Hierdurch wird verhindert, dass es zu einer Irritation des Weichteilgewebes kommt.

An der medialen Seite des proximalen Endes befindet sich bevorzugt eine Nut, über welche die Rotation des Nagels auf dem Zielbügel fixiert wird. Stand der Technik ist demgegenüber die Rotations-Fixierung über zwei Nuten, die jedoch mehr Herstellaufwand mit sich bringen.

Am proximalen Ende befindet sich eine zylindrische Ausnehmung, in welcher der gegengleiche Schaft der Verbindungsschraube zwischen Zielbügel und Nagel eindringen kann. Hierdurch wird die Nagelachse koaxial zum Zielbügel ausgerichtet, wobei das Gewinde nur die Anpresskraft bewirkt. Stand der Technik ist demgegenüber die koaxiale Ausrichtung direkt und einzig über das Gewinde der Verbindungsschraube.

In einer besonderen Ausgestaltungsform ist eine spezielle Ausbildung der Spitze vorgesehen, sodass diese drehgesichert in der Spongiosa im distalen Femurbereich eingeschlagen werden kann, um dort auch ohne Schraubverriegelung drehgesichert zu sein. Die Spitze des Nagels weist in einem Radialschnitt, abweichend von der Kreisform, spezielle Spitzenflächen auf, insbesondere konkave Kerben oder plane Flächen. Bei dieser Variante ist aber ein nachträgliches, willkürliches oder unwillkürliches Rotieren nicht möglich.

Weitere Ausbildungen der Erfindung sind in den Figuren und in den abhängigen Patentansprüchen angegeben.

Diese und die Bezugszeichenliste sind Bestandteil der Offenbarung.

Anhand von Figuren wird die Erfindung symbolisch und beispielhaft näher erläutert.

Die Figuren werden zusammenhängend und übergreifend beschrieben.

Es zeigen dabei
Fig.1 - den erfindungsgemässen Nagel gesehen von anterior nach posterior, also in lateral-medialer Ebene,
Fig.2 - den erfindungsgemässen Nagel gesehen von lateral nach medial, also in anterior-posteriorer Ebene,
Fig.3 - den erfindungsgemässen Nagel gesehen von proximal nach distal,
Fig.4a - eine besondere Ausführungsform der Spitze des erfindungsgemässen Nagels gesehen von lateral nach medial,
Fig.4b - eine besondere Ausführungsform der Spitze des erfindungsgemässen Nagels gesehen von distal nach proximal,
Fig.5a - eine besondere Ausführungsform der Spitze des erfindungsgemässen Nagels gesehen von lateral nach medial,
Fig.5b - eine besondere Ausführungsform der Spitze des erfindungsgemässen Nagels gesehen von distal nach proximal,
Fig.6a - eine besondere Ausführungsform der Spitze des erfindungsgemässen Nagels gesehen von lateral nach medial,
Fig.6b - eine besondere Ausführungsform der Spitze des erfindungsgemässen Nagels gesehen von distal nach proximal und
Fig.7 - das proximale Ende des erfindungsgemässen Nagels gesehen von proximal nach distal.

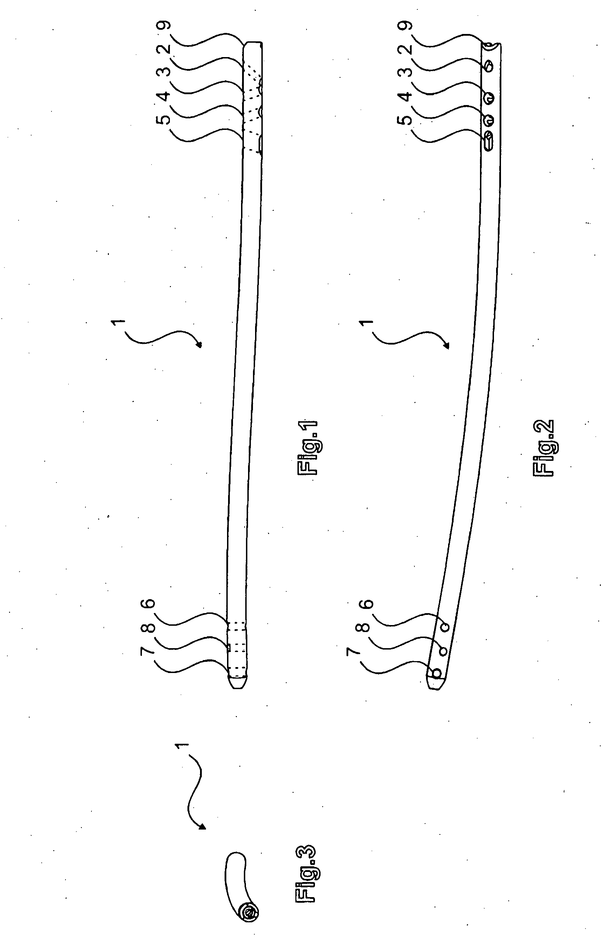

Fig. 1 - 3 stellen den erfindungsgemässen Nagel 1 in drei Ansichten dar. Die proximale und distale Endebene sind 60°-110°, vorzugsweise 70°-90° und insbesondere 80° zu einander verdreht. Der Radius beträgt bei diesem Ausführungsbeispiel 300-1300 mm, vorzugsweise 900-1200 mm und insbesondere ca. 1100 mm. Die Länge des proximalen Radius entspricht der lateralen Kontaktfläche zum Kortex. Sie beträgt 300-1000 mm, vorzugsweise 600-800 mm und insbesondere 700 mm.

Die Länge des distalen geraden Abschnitts entspricht der Tiefe, die das distale Nagelende in die distale Spongiosastruktur eindringt. Sie beträgt 35-70 mm, vorzugsweise 40-60 mm und insbesondere ca. 52 mm.

Der Nagel 1 ist ausgestaltet durch eine 120° antegrade Bohrung 2 für eine 3.9-6.0 mm dicke Verriegelungsschraube, eine kraniale 130° Recon-Bohrung 3 für eine ca. 6.5 mm dicke Schaftschraube, die medial mit einer 120° Antegraden Bohrung 2 für eine 3.9-6.0 mm dicke Verriegelungsschraube zusammenfällt, eine kaudale ca. 130° Recon-Bohrung 4 für eine ca. 6.5 mm dicke Schaftschraube und eine ovale Bohrung 5 für statische und dynamische Positionierung einer 3.9-6.0 mm dicken Verriegelungsschraube im proximalen Bereich. Des weiteren ist am proximalen Ende ein laterale Schräge 9 erkennbar.

Schliesslich sind zwei quer und zueinander parallel verlaufende Bohrungen 6 und 7 und eine anterolaterale Bohrung 8, welche um 25° zu den parallel verlaufenden Bohrungen 6 und 7 rotiert ist, am distalen Ende dargestellt. Der Winkel liegt vorzugsweise zwischen 45° und 10° (0° entspricht der Frontalebene bzw. der Ebene der beiden Standard-Verriegelungsschrauben).

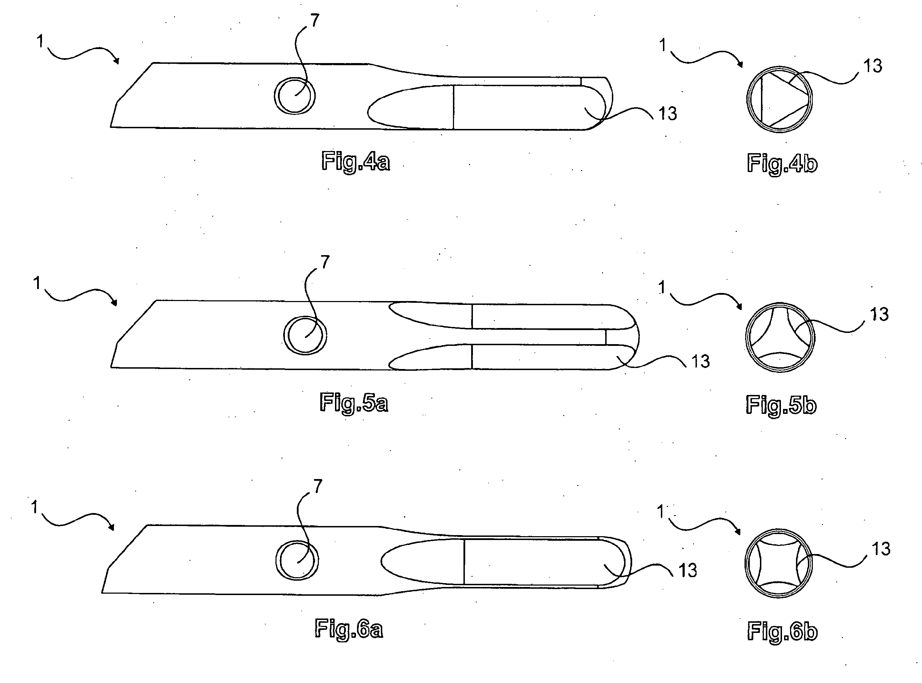

Fig. 4a und 4b stellen eine Variante der Spitze des erfindungsgemässen Nagels 1 in zwei Ansichten dar. Sie weist in einem Radialschnitt, abweichend von der Kreisform spezielle Spitzenflächen 13, insbesondere drei plane Flächen auf, welche eine Länge von 10-40mm, vorzugsweise 15-25mm und insbesondere 20mm aufweisen. Die Gesamtlänge der Spitze beträgt 20-50mm, vorzugsweise 25-35mm und insbesondere 30mm. Zusätzlich ist die Bohrung 7 dargestellt.

Fig. 5a und 5b stellen eine Variante der Spitze des erfindungsgemässen Nagels 1 in zwei Ansichten dar. Sie weist in einem Radialschnitt, abweichend von der Kreisform spezielle Spitzenflächen 13, insbesondere drei konkave Kerben auf, welche eine Länge von 10-40mm, vorzugsweise 15-25mm und insbesondere 20mm und einen Radius von 4-10mm, vorzugsweise 5-8mm und insbesondere 6mm aufweisen. Die Gesamtlänge der Spitze beträgt 20-50mm, vorzugsweise 25-35mm und insbesondere 30mm. Zusätzlich ist die Bohrung 7 dargestellt.

Fig. 6a und 6b stellen eine Variante der Spitze des erfindungsgemässen Nagels 1 in zwei Ansichten dar. Sie weist in einem Radialschnitt, abweichend von der Kreisform spezielle Spitzenflächen 13, insbesondere vier konkave Kerben auf, welche eine Länge von 10-40mm, vorzugsweise 15-25mm und insbesondere ca. 20mm und einen Radius von 4-10mm, vorzugsweise 5-8mm und insbesondere ca. 6mm aufweisen. Die Gesamtlänge der Spitze beträgt 20-50mm, vorzugsweise 25-35mm und insbesondere ca. 30mm. Zusätzlich ist die Bohrung 7 dargestellt.

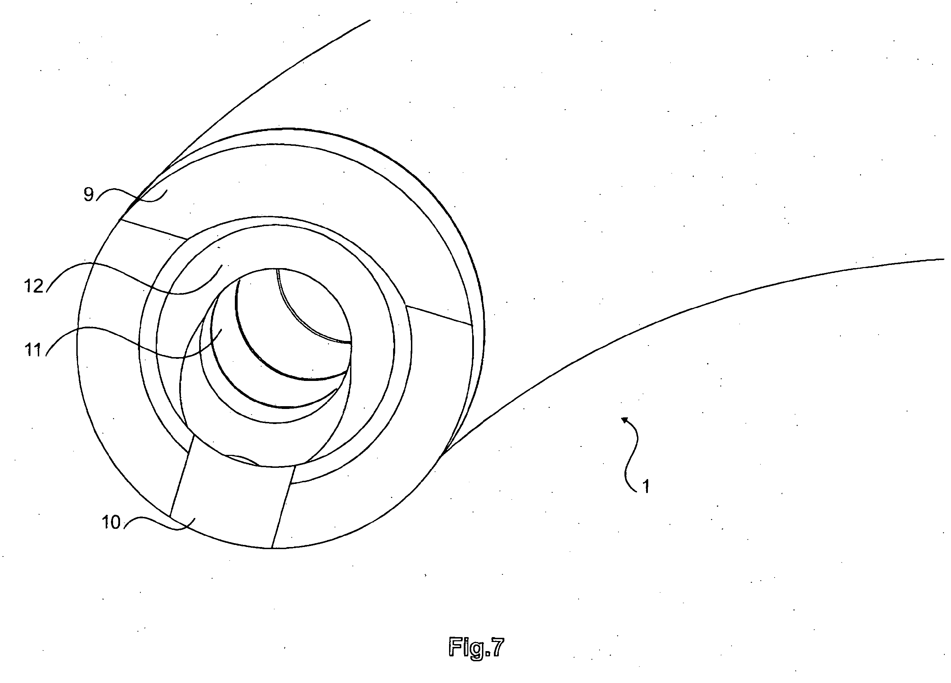

Fig. 7 stellt das proximale Ende des erfindungsgemässen Nagels 1 dar, gesehen von proximal nach distal. Lateral ist eine Schräge 9 dargestellt, welche einen Winkel am lateral-proximalen Ende zur axialen Nagelachse von 10° bis 60°, vorzugsweise ca. 40° aufweist. Schliesslich ist eine zylindrische Ausnehmung 12 mit einem Gewinde 11, und an der medialen Seite des proximalen Endes eine Positionierungsnut 10 dargestellt.

### Bezugszeichenliste

1 - Marknagel
2 - antegrade Bohrung
3 - kraniale Bohrung
4 - kaudale Bohrung
5 - ovale Bohrung
6 - zu 7 parallele Bohrung
7 - zu 6 parallele Bohrung
8 - anterolaterale Bohrung
9 - laterale Schräge
10 - Positionierungsnut
11 - Gewinde
12 - zylindrische Ausnehmung
13 - Spitzenfläche

## Patentansprüche

1. Marknagel (1) zur Einführung in den Markraum eines Femur durch die laterale Kompakta des Trochanter Major, welcher einen proximalen Nagelabschnitt mit einer ersten Endebene und einen geraden distalen Nagelabschnitt mit einer zweiten Endebene, sowie Bohrungen für Knochenschrauben in beiden Nagelabschnitten aufweist, wobei der proximale Nagelabschnitt mit mindestens einer, schräg zu einer Längsachse des Marknagels (1) verlaufenden Bohrung zur Einführung der Knochenschraube in den Femurkopf versehen ist, **dadurch gekennzeichnet, dass** die erste Endebene des proximalen Nagelabschnittes und die zweite Endebene des distalen Nagelabschnittes zueinander um 60°-110°, vorzugsweise um 80° verdreht angeordnet sind, und dass der proximale Nagelabschnitt eine Krümmung in einer Ebene, namentlich in lateralposteriorer Richtung, im implantierten Zustand, aufweist, deren Radius 300-1300 mm, vorzugsweise 900-1200 mm, insbesondere 1100 mm beträgt.

2. Marknagel nach Anspruch 1, **dadurch gekennzeichnet, dass** eine der Bohrungen im proximalen Nagelabschnitt als eine antegrade Bohrung (2), vorzugsweise in 120° zur Längsachse ausgebildet ist

3. Marknagel nach Anspruch 1 oder 2, **dadurch gekennzeichnet, dass** eine Länge des proximalen Nagelabschnittes der lateralen Kontaktfläche zum Kortex entspricht.

4. Marknagel nach einem der vorhergehenden Ansprüche, **dadurch gekennzeichnet, dass** die Länge des distalen geraden Nagelabschnitts einer Tiefe entspricht, bei der das Ende des distalen Nagelabschnittes im implantierten Zustand in die distale Spongiosastruktur eindringt, und diese Länge 35-70 mm, vorzugsweise 52 mm beträgt

5. Marknagel nach einem der vorhergehenden Ansprüche, **dadurch gekennzeichnet, dass** der Nagel (1) so ausgebildet ist, dass er beim Erreichen seines Endimplantationstiefenbereichs aufhört, von selbst zu rotieren, und im Endimplantationstiefenbereich ohne Rotation entlang seiner Längsachse verschoben werden kann und/oder ohne Änderung seiner Implantationstiefe um seine Längsachse rotierbar ist

6. Marknagel nach einem der vorhergehenden Ansprüche, **dadurch gekennzeichnet, dass** der Nagel (1) folgende Bohrungen aufweist:
▪ eine 120° antegrade Bohrung (2) für eine ca. 5.0 mm dicke Verriegelungsschraube;
▪ eine kraniale 130° Recon-Bohrung (3) für eine ca. 6.5 mm dicke Schaftschraube, die medial mit der antegraden Bohrung (2) zusammenfällt;
▪ eine kaudale 130° Recon-Bohrung (4) für eine ca. 3.9-6.0 mm dicke Schaftschraube;
▪ eine ovale Bohrung (5) für statische und dynamische Positionierung einer ca. 3.9-6.0 mm dicken Verriegelungsschraube im proximalen Nagelabschnitt;
▪ zwei quer und zueinander parallel verlaufende Bohrungen (6, 7) und eine zu diesen Bohrungen (6, 7) um 25° rotierte antero-laterale Bohrung (8) im distalen Nagelabschnitt

7. Marknagel nach einem der vorhergehenden Ansprüche, **dadurch gekennzeichnet, dass** der proximale Nagelabschnitt eine ovale Bohrung (5) aufweist.

8. Marknagel nach einem der vorhergehenden Ansprüche, **dadurch gekennzeichnet, dass** der Marknagel (1) an seinem proximale Nagelabschnitt eine laterale Schräge (9) aufweist, welche einen Winkel am lateral-proximalen Ende zur Längsachse des Marknagels (1) von 10° bis 60°, vorzugsweise 40° aufweist.

9. Marknagel nach einem der vorhergehenden Ansprüche, **dadurch gekennzeichnet, dass** der Marknagel (1) am Ende des proximalen Nagelabschnittes eine einzige Positionierungsnut (10) aufweist.

10. Marknagel nach einem der vorhergehenden Ansprüche, **dadurch gekennzeichnet, dass** der Marknagel (1) am Ende des proximalen Nagelabschnittes eine zylindrische Ausnehmung (12) mit einem Gewinde (11) aufweist.

11. Marknagel nach Anspruch 10, **dadurch gekennzeichnet, dass** die Längsachse des Marknagels (1) durch die zylindrische Ausnehmung (12) koaxial zu einem Zielbügel ausrichtbar ist und das Gewinde (11) im die Anpresskraft bewirkt.

12. Marknagel nach einem der vorhergehenden Ansprüche, **dadurch gekennzeichnet, dass** die Gesamtlänge des distalen Nagelabschnittes 20-50 mm, vorzugsweise 25-35 mm, insbesondere 30 mm beträgt, und dass die Spitz des distalen Nagelabschnitts in elnem Radialschnitt, von der Kreisform abweichende Spitzenflächen (13), insbesondere mindestens drei konkave Kerben aufweist, deren Länge 10-40 mm, vorzugsweise 15-25 mm, insbesondere 20 mm, sowie deren Radius 4-10 mm, vorzugsweise 5-8 mm, insbesondere 6 mm beträgt.

13. Marknagel nach einem der Ansprüche 1-11, **dadurch gekennzeichnet, dass** die Gesamtlänge des distalen Nagelabschnittes 20-50 mm, vorzugsweise 25-35 mm, insbesondere 30 mm beträgt, und dass die Spitz des distalen Nagelabschnitts in einem Radialschnitt von der Kreisform abweichende Spitzenflächen (13), insbesondere mindestens drei plane Flächen aufweist, deren Länge 10-40 mm, vorzugsweise 15-25 mm, insbesondere 20 mm beträgt.

14. Marknagel nach einem der vorhergehenden Ansprüche, **dadurch gekennzeichnet, dass** die Spitze des distalen Nagelabschnittes derart ausgebildet ist, dass sie drehgesichert in die Spongiosa im distalen Femurbereich einschlagbar ist, um dort auch ohne Schraubverriegelung drehgesichert zu sein.

## Claims

1. An intramedullary nail (1) for insertion into a medullary space of a femur through the lateral compacta of the trochanter major, comprising a proximal nail section having a first end plane and a straight distal nail section having a distal end plane, and bores for bone screws in both nail sections, wherein the proximal nail section has at least one bore running obliquely to the longitudinal axis of the intramedullary nail (1) for insertion of the bone screw into the head of the femur, **characterized in that** the first end plane of the proximal nail section and the second end plane of the distal nail section are rotated about 60° to 110°, preferably 80°, relative to one another, and **in that** the proximal nail section has, in an implanted state, a curvature in a plane, namely in the lateral-posterior direction, the radius of which is 300-1300 mm, preferably 900-1200 mm, particularly 1100 mm.

2. An intramedullary nail according to claim 1, **characterized in that** one of the bores in the proximal nail section is formed as an antegrade bore (2), preferably in 120° to the longitudinal axis.

3. An intramedullary nail according to claim 1 or 2, **characterized in that** the length of the proximal nail section corresponds to a lateral contact surface with the cortex.

4. An intramedullary nail according to any one of the preceding claims, **characterized in that** the length of the distal straight nail section corresponds to a depth at which the end of the distal nail section in an implanted state penetrates into the distal spongiosa structure and this length is 35-70 mm, preferably 52 mm.

5. An intramedullary nail according to any one of the preceding claims, **characterized in that** the nail (1) is formed in such a way that, on reaching its final implantation depth range, the nail ceases to rotate by itself and, in the final implantation depth range, can be displaced along its longitudinal axis without rotation, and/or can be rotated about its longitudinal axis without changing its implantation depth.

6. An intramedullary nail according to any one of the preceding claims, **characterized in that** the nail (1) has the following bores:
- a 120° antegrade bore (2) for a locking screw about 5.0 mm thick,
- a cranial 130° recon bore (3) for a headless screw about 6.5 mm thick, medially coinciding with the antegrade bore (2),
- a caudal 130° recon bore (4) for a headless screw about 3.9-6.0 mm thick,
- an oval bore (5) for static and dynamic positioning of a locking screw about 3.9-6.0 mm thick in the proximal nail section,
- two bores (6, 7) transverse and parallel to one another, and an anterolateral bore (8) rotated through 25° relative to the these bores (6, 7) in the distal nail section.

7. An intramedullary nail according to any one of the preceding claims, **characterized in that** the proximal nail section has an oval bore (5).

8. An intramedullary nail according to any one of the preceding claims, **characterized in that** the intramedullary nail (1) has at its proximal nail section a lateral bevel (9) having an angle of 10° to 60°, preferably 40°, to the longitudinal axis of the intramedullary nail (1) at the lateral-proximal end.

9. An intramedullary nail according to any one of the preceding claims, **characterized in that** the intramedullary nail (1) has, at the end of the proximal nail section, a single positioning groove (10).

10. An intramedullary nail according to any one of the preceding claims, **characterized in that** the intramedullary nail (1) has, at the end of the proximal nail section, a cylindrical recess (12) with a thread (11).

11. An intramedullary nail according to claim 10, **characterized in that** the longitudinal axis of the intramedullary nail (1) through the cylindrical recess (12) is alignable coaxially with a target bow, and the thread (11) exerts only contact
pressing force.

12. An intramedullary nail according to any one of the preceding claims, **characterized in that** the total length of the distal nail section is 20-50 mm, preferably 25-35 mm, particularly 30 mm, and that the tip of the distal nail section has in a radial section tip surfaces (13) differing from the circular shape, particularly at least three concave notches having a length of 10-40 mm, preferably 15-25 mm, particularly 20 mm, and a radius of 4-10 mm, preferably 5-8 mm, particularly 6 mm.

13. An intramedullary nail according to any one of claims 1 to 11, **characterized in that** the total length of the distal nail section is 20-50 mm, preferably 25-35 mm, particularly 30 mm, and that the tip of the distal nail section has in a radial section tip surfaces (13) differing from the circular shape, particularly at least three planar surfaces, having a length of 10-40 mm, preferably 15-25 mm, particularly 20 mm.

14. An intramedullary nail according to any one of the preceding claims, **characterized in that** the tip of the distal nail section is formed in such a way that it can be tapped rotatably locked into the spongiosa in the distal femur region, so as to be there rotatably locked even without screw locking.

## Revendications

1. Clou intramédullaire (1) destiné à être introduit dans le canal médullaire d'un fémur par l'os compact latéral du grand trochanter, lequel présente une partie de clou proximale avec un premier plan d'extrémité et une partie de clou distale droite avec un second plan d'extrémité, ainsi que des alésages pour vis à os dans les deux parties de clou, la partie de clou proximale étant prévue avec au moins un alésage s'étendant de manière oblique par rapport à un axe longitudinal du clou intramédullaire (1) en vue d'une introduction de la vis à os dans la tête de fémur, **caractérisé en ce que** le premier plan d'extrémité de la partie de clou proximale et le second plan d'extrémité de la partie de clou distale sont orientés l'un vers l'autre suivant une torsion de 60°-110°, de préférence de 80°, et **en ce que** la partie de clou proximale présente une courbure dans un plan, à savoir dans la direction latérale/postérieure, à l'état implanté, dont le rayon s'élève à 300-1300 mm, de préférence 900-1200 mm, et en particulier 1100 mm.

2. Clou intramédullaire selon la revendication 1, **caractérisé en ce qu'**un des alésages est formé dans la partie de clou proximale comme alésage antérograde (2), de préférence à 120° par rapport à l'axe longitudinal.

3. Clou intramédullaire selon la revendication 1 ou 2, **caractérisé en ce qu'**une longueur de la partie de clou proximale correspond à la surface de contact latérale avec le cortex.

4. Clou intramédullaire selon l'une quelconque des revendications précédentes, **caractérisé en ce que** la longueur de la partie de clou distale droite correspond à une profondeur, à laquelle l'extrémité de la partie de clou distale pénètre à l'état implanté dans la structure spongieuse distale, et ladite longueur est de 35-70 mm, de préférence de 52 mm.

5. Clou intramédullaire selon l'une quelconque des revendications précédentes, **caractérisé en ce que** le clou (1) est façonné de telle sorte qu'une fois sa plage de profondeur d'implantation finale atteinte, il arrête de tourner par lui-même et peut être glissé, dans la plage de profondeur d'implantation finale, sans rotation, le long d'un axe longitudinal, et/ou il peut tourner autour de son axe longitudinal, sans modification de sa profondeur d'implantation.

6. Clou intramédullaire selon l'une quelconque des revendications précédentes, **caractérisé en ce que** le clou (1) présente les alésages suivants :
• un alésage antérograde de 120° (2) pour une vis de verrouillage d'une épaisseur d'environ 5,0 mm ;
• un alésage Recon crânial de 130° (3) pour une vis sans tête d'une épaisseur d'environ 6,5 mm, lequel coïncide de manière médiale avec l'alésage antérograde (2) ;
• un alésage Recon caudal de 130° (4) pour une vis sans tête d'une épaisseur d'environ 3,9-6,0 mm ;
• un alésage ovale (5) pour un positionnement statique et dynamique d'une vis de verrouillage d'une épaisseur d'environ 3,9-6,0 mm dans la partie de clou proximale ;
• deux alésages s'étendant transversalement et parallèlement l'un par rapport à l'autre (6, 7) et un alésage (8) antéro-latéral tourné à 25° vers lesdits alésages (6,7) dans la partie de clou distale.

7. Clou intramédullaire selon l'une quelconque des revendications précédentes, **caractérisé en ce que** la partie de clou proximale présente un alésage ovale (5).

8. Clou intramédullaire selon l'une quelconque des revendications précédentes, **caractérisé en ce que** le clou intramédullaire (1) présente sur sa partie de clou proximale un biseau latéral (9), lequel présente un angle au niveau de l'extrémité latérale/proximale par rapport à l'axe longitudinal du clou intramédullaire (1) de 10° à 60°, et de préférence de 40°.

9. Clou intramédullaire selon l'une quelconque des revendications précédentes, **caractérisé en ce que** le clou intramédullaire (1) présente une rainure de positionnement unique (10) au niveau de l'extrémité de la partie de clou proximale.

10. Clou intramédullaire selon l'une quelconque des revendications précédentes, **caractérisé en ce que** le clou intramédullaire (1) présente un évidement cylindrique (12) avec un filet (11) au niveau de l'extrémité de la partie de clou proximale.

11. Clou intramédullaire selon la revendication 10, **caractérisé en ce que** l'axe longitudinal du clou intramédullaire (1) peut être aligné par le biais de l'évidement cylindrique (12) coaxialement à un étrier de visée, et le filet (11) n'exerce que la force de pression.

12. Clou intramédullaire selon l'une quelconque des revendications précédentes, **caractérisé en ce que** la longueur totale de la partie de clou distale est de 20-50 mm, de préférence de 25-35 mm, et en particulier de 30 mm, et **en ce que** la pointe de la partie de clou distale présente, dans une coupe radiale, des surfaces de pointe différant de la forme ronde (13), en particulier au moins trois encoches concaves dont la longueur est de 10-40 mm, de préférence 15-25 mm, et en particulier de 20 mm, et dont le rayon est de 4-10 mm, de préférence de 5-8 mm, et en particulier de 6 mm.

13. Clou intramédullaire selon l'une quelconque des revendications 1 à 11, **caractérisé en ce que** la longueur totale de la partie de clou distale est de 20-50 mm, de préférence de 25-35 mm, et en particulier de 30 mm, et **en ce que** la pointe de la partie de clou distale présente, dans une coupe radiale, des surfaces de pointe différant de la forme ronde (13), en particulier au moins trois surfaces planes (13) dont la longueur est de 10-40 mm, de préférence 15-25 mm, et en particulier de 20 mm.

14. Clou intramédullaire selon l'une quelconque des revendications précédentes, **caractérisé en ce que** la pointe de la partie de clou distale est façonnée de telle sorte qu'elle peut être enfoncée, de manière fixe en rotation, dans l'os spongieux dans la plage de fémur distale, afin de se trouver à cet endroit également sans vis de verrouillage et de manière fixe en rotation.
